(19) Europäisches Patentamt
European Patent Office
Office européen des brevets

(11) EP 2 047 906 B1

(12) EUROPEAN PATENT SPECIFICATION

(45) Date of publication and mention of the grant of the patent:
26.08.2015 Bulletin 2015/35

(51) Int Cl.:
$B01J\ 29/74^{(2006.01)}$    $B01J\ 35/10^{(2006.01)}$
$C07C\ 5/27^{(2006.01)}$

(21) Application number: 08253097.3

(22) Date of filing: 23.09.2008

(54) **Aromatic isomerization catalyst**

Aromatischer Isomerisierungskatalysator

Catalyseur d'isomérisation aromatique

(84) Designated Contracting States:
FR PT TR

(30) Priority: 08.10.2007 US 868844

(43) Date of publication of application:
15.04.2009 Bulletin 2009/16

(73) Proprietor: UOP LLC
Des Plaines, IL 60017-5017 (US)

(72) Inventors:
• Whitchurch, Patrick Charles
Shreveport, LA 71107 (US)
• Bogdan, Paula Lucy
Des Plaines, IL 60017-5017 (US)
• Bauer, John Edward
Des Plaines, IL 60017-5017 (US)

(74) Representative: Boult Wade Tennant
Verulam Gardens
70 Gray's Inn Road
London WC1X 8BT (GB)

(56) References cited:
EP-A- 0 135 658          US-A- 5 081 084
US-A1- 2004 097 363    US-A1- 2005 143 615

• NAYAK V S ET AL: "ISOMERIZATION OF m-XYLENE ON H-ZSM-5 - 1. INFLUENCE ON CATALYTIC ACTIVITY AND SELECTIVITY OF Si/AI RATIO, DEGREE OF CATION EXCHANGE, DEAMMONIATION CONDITIONS AND POISONING OF STRONGER ACID SITES." APPLIED CATALYSIS 1982 DEC 15, vol. 4, no. 4, 15 December 1982 (1982-12-15), pages 333-352, XP002511424

Remarks:
The file contains technical information submitted after the application was filed and not included in this specification

EP 2 047 906 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The field of this invention generally relates to a catalyst for a C8 aromatic isomerization process or zone.

BACKGROUND OF THE INVENTION

The xylenes, such as para-xylene, meta-xylene and ortho-xylene, can be important intermediates that find wide and varied application in chemical syntheses. Generally, paraxylene upon oxidation yields terephthalic acid that is used in the manufacture of synthetic textile fibers and resins. Meta-xylene can be used in the manufacture of plasticizers, azo dyes, wood preservers, etc. Generally, ortho-xylene is feedstock for phthalic anhydride production.

Xylene isomers from catalytic reforming or other sources generally do not match demand proportions as chemical intermediates, and further comprise ethylbenzene, which can be difficult to separate or to convert. Typically, para-xylene is a major chemical intermediate with significant demand, but amounts to only 20-25% of a typical C8 aromatic stream. Adjustment of an isomer ratio to demand can be effected by combining xylene-isomer recovery, such as adsorption for para-xylene recovery, with isomerization to yield an additional quantity of the desired isomer. Typically, isomerization converts a non-equilibrium mixture of the xylene isomers that is lean in the desired xylene isomer to a mixture approaching equilibrium concentrations.

Various catalysts and processes have been developed to effect xylene isomerization. In selecting appropriate technology, it is desirable to run the isomerization process as close to equilibrium as practical in order to maximize the para-xylene yield; however, associated with this is a greater cyclic C8 loss due to side reactions. Often, the approach to equilibrium that is used is an optimized compromise between high C8 cyclic loss at high conversion (i.e., very close approach to equilibrium) and high utility costs due to the large recycle rate of unconverted C8 aromatic. Thus, catalysts can be evaluated on the basis of a favorable balance of activity, selectivity and stability.

Such catalysts can include binders of alumina. The source of alumina and the various processing steps used to make the catalysts result in different physical and chemical properties and the impact on catalyst performance may be unpredictable. Suitable sources of alumina binders may include aluminum hydroxychloride hydrosol typically used in an oil dropped sphere (ODS alumina), or Bohemite typically used in extruded catalysts. An oil dropped gamma sphere may tend to have less C8 ring loss as compared to a catalyst with an extruded gamma-alumina. Generally, it is desirable to produce an extruded gamma-alumina with similar performance because such a catalyst can be less expensive than catalyst with a binder of ODS alumina. Thus, a catalyst that can isomerize ethylbenzene to xylenes while minimizing C8 ring loss would be beneficial.

US 2005/0143615 discloses a process for isomerizing ethylbenzene into xylenes such as para-xylene using a bimetallic zeolite catalyst system.

BRIEF SUMMARY OF THE INVENTION

In the first aspect there is provided a catalyst as defined in claim 1.

Another disclosure is a catalyst for a C8 aromatic isomerization process. The catalyst can include: 1 - 90%, by weight, of a zeolite including an MTW zeolite; 10 - 99%, by weight, of a binder including a gamma-alumina, the gamma-alumina being derived from a Boehmite alumina; 0.1 - 2%, by weight, of a noble metal, calculated on an elemental basis; and at least one alkali metal. A total alkali metal content of the catalyst can be at least 100 ppm, by weight, calculated on an elemental basis. Generally, the catalyst has a pore volume distribution and at least 70% of a pore volume of the catalyst is defined by pores having a diameter greater than 100 Å (not within the scope of the invention).

Another disclosure is a catalyst for a C8 aromatic isomerization process. The catalyst can include: 1 - 90%, by weight, of a zeolite including an MTW zeolite; 10 - 99%, by weight, of a binder including a gamma-alumina; 0.1 - 2%, by weight, of a noble metal, calculated on an elemental basis; and at least one alkali metal wherein a total alkali metal content of the catalyst is at least 100 ppm, by weight, calculated on an elemental basis. Generally, the catalyst has a pore volume distribution and at least 70% of a pore volume of the catalyst is defined by pores having a diameter greater than 100 Å and at least 95% of the pore volume of the catalyst is defined by pores having a diameter less than 1000 Å (not within the scope of the invention).

A further disclosure is an aromatic production facility. The aromatic production facility can include an xylene isomer separation zone, and a C8 aromatic isomerization zone receiving a stream depleted of at least one xylene isomer from the xylenes isomer separation zone. Generally, the C8 aromatic isomerization zone includes a catalyst having a pore volume distribution and at least 70% of a pore volume of the catalyst is defined by pores having a diameter greater than 100 Å. The catalyst may include: 1 - 90%, by weight, of a zeolite including an MTW zeolite; 10 - 99%, by weight, of a binder including a gamma-alumina; 0.1 - 2%, by weight, of a noble metal, calculated on an elemental basis; and at least one alkali metal. A total alkali metal content of the catalyst can be at least 100 ppm, by weight, calculated on an elemental basis (not according to the invention).

Therefore, the catalyst can provide lower C8 ring loss to levels at or below a catalyst made with a binder of an ODS alumina. In some exemplary embodiments, it has been found that utilizing an alumina with a defined pore size distribution and a higher alkali metal content by, e.g., omitting a catalyst wash, can yield a catalyst with a low C8 ring loss.

BRIEF DESCRIPTION OF THE DRAWINGS

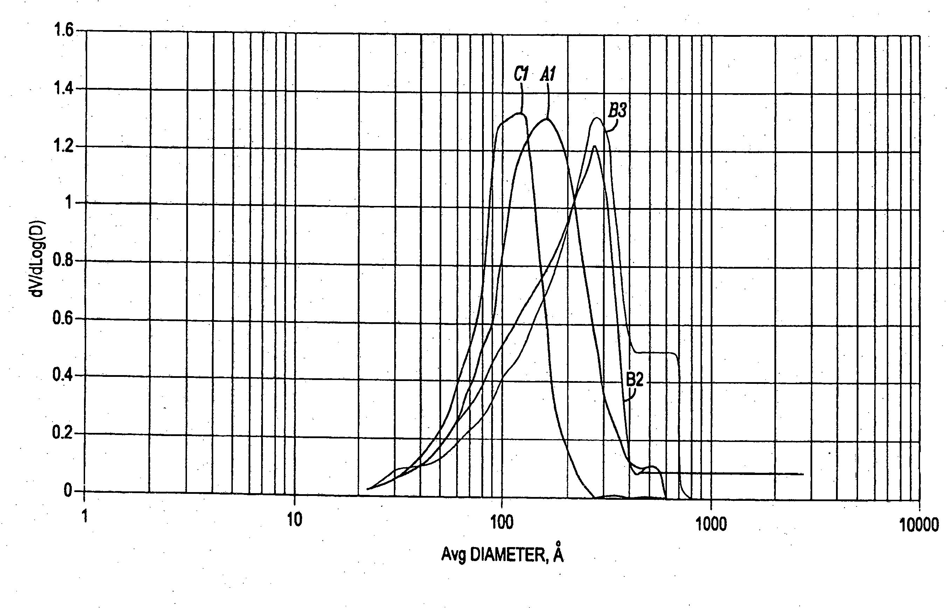

FIG. 1 is a graphical depiction of pore volume distribution of several impregnated, oxidated, and reduced catalysts.

DEFINITIONS

As used herein, the term "zone" can refer to an area including one or more equipment items and/or one or more sub-zones. Equipment items can include one or more reactors or reactor vessels, heaters, separators, exchangers, pipes, pumps, compressors, and controllers. Additionally, an equipment item, such as a reactor or vessel, can further include one or more zones or sub-zones.

As used herein, the term "stream" can be a stream including various hydrocarbon molecules, such as straight-chain, branched, or cyclic alkanes, alkenes, alkadienes, and alkynes, and optionally other substances, such as gases, e.g., hydrogen, or impurities, such as heavy metals. The stream can also include aromatic and non-aromatic hydrocarbons. Moreover, the hydrocarbon molecules may be abbreviated C1, C2, C3 ... Cn where "n" represents the number of carbon atoms in the hydrocarbon molecule.

As used herein, the term "aromatic" can mean a group containing one or more rings of unsaturated cyclic carbon radicals where one or more of the carbon radicals can be replaced by one or more non-carbon radicals. An exemplary aromatic compound is benzene having a C6 ring containing three double bonds. Other exemplary aromatic compounds can include para-xylene, ortho-xylene, meta-xylene and ethylbenzene. Moreover, characterizing a stream or zone as "aromatic" can imply one or more different aromatic compounds.

As used herein, the term "support" generally means a molecular sieve that has been combined with a binder before the addition of one or more additional catalytically active components, such as a noble metal, or a subsequent process such as reducing or sulfiding.

DETAILED DESCRIPTION OF THE INVENTION

Generally, a refinery or a petrochemical production facility can include an aromatic production facility or an aromatic complex, particularly a C8 aromatic complex that purifies a reformate to extract one or more xylene isomers, such as para-xylene or meta-xylene. Such an aromatic complex for extracting para-xylene is disclosed in US 6,740,788 B1. A feedstock to an aromatic complex can include an isomerizable C8 aromatic hydrocarbon of the general formula $C_6H_{(6-n)}R_n$, where n is an integer from 2 to 5 and R is $CH_3$, $C_2H_5$, $C_3H_7$, or $C_4H_9$, in any combination and including all the isomers thereof. Suitable C8 aromatic hydrocarbons may include ortho-xylene, meta-xylene, para-xylene, ethylbenzene, ethyltoluene, tri-methylbenzene, di-ethylbenzene, tri-ethylbenzene, methylpropylbenzene, ethylpropylbenzene, di-isopropylbenzene, or a mixture thereof.

An aromatic complex can include a xylene isomer separation zone, such as a paraxylene separation zone, and a C8 aromatic isomerization zone. The C8 aromatic isomerization zone can receive a stream depleted of at least one xylene isomer, such as paraxylene or meta-xylene. The C8 aromatic isomerization zone can reestablish the equilibrium concentration of xylene isomers and convert other compounds, such as ethylbenzene, into a xylene. Typically, such a zone can increase the amount of a xylene isomer, such as paraxylene, and the product from that C8 aromatic isomerization zone can be recycled to the xylene isomer separation zone to recover more of the desired isomer.

One exemplary application of the catalyst disclosed herein is the isomerization of a $C_8$ aromatic mixture containing ethylbenzene and xylenes. Generally, the mixture has an ethylbenzene content of 1 - 50 %, by weight, an ortho-xylene content of up to 35 %, by weight, a meta-xylene content of 20 - 95 %, by weight, and a para-xylene content of up to 30 %, by weight. The aforementioned C8 aromatics are a non-equilibrium mixture, i.e., at least one C8 aromatic isomer is present in a concentration that differs substantially from the equilibrium concentration at isomerization conditions. Usually the non-equilibrium mixture is prepared by removal of para-, ortho- and/or meta-xylene from a fresh C8 aromatic mixture obtained from an aromatic production process.

Accordingly, a C8 aromatic hydrocarbon feed mixture, preferably in admixture with hydrogen, can be contacted with a catalyst hereinafter described in an C8 aromatic hydrocarbon isomerization zone. Contacting may be effected using the catalyst in a fixed bed system, a moving bed system, a fluidized bed system, or in a batch operation. Preferably, a fixed bed system is utilized. In this system, a hydrogen-rich gas and the feed mixture are preheated by any suitable heating means to the desired reaction temperature and then passed into a C8 aromatic isomerization zone containing a fixed bed of catalyst. The conversion zone may be one or more separate reactors with suitable means therebetween to ensure that the desired isomerization temperature is maintained at the entrance of each zone. The reactants may be contacted with the catalyst bed in either upward-, downward-, or radial-flow fashion, and the reactants may be in the liquid phase, a mixed liquid-vapor phase, or a vapor phase when contacted with the catalyst.

The feed mixture, preferably a non-equilibrium mixture of C8 aromatics, may be contacted with the isomerization catalyst at suitable C8 isomerization conditions. Generally, such conditions include a temperature ranging from 0 -600° C. or more, preferably 300 - 500°C. Generally, the pressure is from 100 - 10,000 kPa absolute, preferably less than 5,000 kPa. Sufficient catalyst may be contained in the isomerization zone to provide a liquid hourly space velocity with respect to the hydrocarbon feed mixture of from 0.1 - 30 $h^{-1}$, and preferably 0.5 - 10 $hr^{-1}$. The hydrocarbon feed mixture can be reacted in admixture with hydrogen at a hydrogen/hydrocarbon mole ratio of 0.5:1 - 25:1 or more. Other inert diluents such as nitrogen, argon and light hydrocarbons may be present.

The reaction can isomerize xylenes while reacting ethylbenzene to form a xylene mixture via conversion to and reconversion from naphthenes. Thus, the yield of xylenes in the product may be enhanced by forming xylenes from ethylbenzene. Typically, the loss of C8 aromatics through the reaction is low, generally less than 4%, by mole, preferably no more than 3.5%, by mole, and most preferably less than 3 %, by mole, per pass of C8 aromatics in the feed to the reactor.

Any effective recovery scheme may be used to recover an isomerized product from the effluent of the reactors. Typically, the liquid product is fractionated to remove light and/or heavy byproducts to obtain the isomerized product. Heavy byproducts can include aromatic C 10 compounds such as dimethylethylbenzene. In some instances, certain product species such as ortho-xylene or dimethylethylbenzene may be recovered from the isomerized product by selective fractionation. The product from isomerization of C8 aromatics usually is processed to selectively recover the para-xylene isomer, optionally by crystallization. Selective adsorption can be accomplished by using crystalline aluminosilicates according to US 3,201,491.

A catalyst of the C8 aromatic isomerization zone can include at least one MTW zeolitic molecular sieve, also characterized as "low silica ZSM-12" and can include molecular sieves with a silica to alumina ratio less than 45, preferably from 20 - 40. Preferably, the MTW zeolite is substantially mordenite-free, which generally means an MTW component containing less than 20 %, by weight, mordenite impurity, preferably less than 10 %, by weight, and most preferably less than 5 %, by weight, mordenite.

The preparation of an MTW zeolite by crystallizing a mixture including an alumina source, a silica source and a templating agent is known. US 3,832,449 discloses an MTW zeolite using tetraalkylammonium cations. US 4,452,769 and US 4,537,758 disclose a methyltriethylammonium cation to prepare a highly siliceous MTW zeolite. US 6,652,832 uses an N,N-dimethylhexamethyleneimine cation as a template to produce low silica-to-alumina ratio MTW zeolite without MFI impurities. Preferably high purity crystals are used as seeds for subsequent batches.

The MTW zeolite is composited with a binder for convenient formation of particles. The proportion of zeolite in the catalyst is 1 - 10%, by weight, and optimally 5 - 10%, by weight. Generally, it is desirable for the MTW zeolite to contain 0.3 - 0.5%, by weight, $Na_2O$ and 0.3 - 0.5%, by weight, $K_2O$. Also, in one exemplary embodiment it is desirable for the molar ratio of silica to alumina to be 36:1 and the molar ratio of (Na + K)/Al to be 0.2 - 0.3.

The zeolite is combined with a refractory inorganic oxide binder. The binder should be a porous, adsorptive support having a surface area of 25 - 500 $m^2/g$, preferably 200 -500 $m^2/g$. The inorganic oxide is a gamma-alumina. Such a gamma-alumina is derived from a Boehmite alumina. The Boehmite alumina can be compounded with the zeolite and extruded. During oxidation (or calcination), the Boehmite alumina may be converted into gamma-alumina. One desired Beohmite alumina utilized as a starting material is VERSAL-251 sold by UOP, LLC of Des Plaines, IL. The catalyst has 10 - 99%, by weight, desirably 90 - 99%, by weight, of the gamma-alumina binder.

One shape for the support or catalyst can be an extrudate. Generally, the extrusion initially involves mixing of the molecular sieve with optionally the binder and a suitable peptizing agent to form a homogeneous dough or thick paste having the correct moisture content to allow for the formation of extrudates with acceptable integrity to withstand direct calcination. Extrudability may be determined from an analysis of the moisture content of the dough, with a moisture content in the range of from 30 - 70%, by weight, being preferred. The dough may then be extruded through a die pierced with multiple holes and the spaghetti-shaped extrudate can be cut to form particles in accordance with known techniques. A multitude of different extrudate shapes is possible, including a cylinder, cloverleaf, dumbbell, and symmetrical and asymmetrical polylobates. Furthermore, the dough or extrudates may be shaped to any desired form, such as a sphere, by, e.g., marumerization that can entail one or more moving plates or compressing the dough or extrudate into molds.

Alternatively, support or catalyst pellets can be formed into spherical particles by accretion methods. Such a method can entail adding liquid to a powder mixture of zeolite and binder in a rotating pan or conical vessel having a rotating auger.

Generally, preparation of alumina-bound spheres involves dropping a mixture of molecular sieve, alsol, and gelling agent into an oil bath maintained at elevated temperatures. Examples of gelling agents that may be used in this process include hexamethylene tetraamine, urea, and mixtures thereof. The gelling agents can release ammonia at the elevated temperatures which sets or converts the hydrosol spheres into hydrogel spheres. The spheres may be then withdrawn from the oil bath and typically subjected to specific aging treatments in oil and an ammonia solution to further improve their physical characteristics. One exemplary oil dropping method is disclosed in US 2,620,314 .

Generally, the subsequent drying, calcining, and optional washing steps can be done before and/or after impregnation with one or more components, such as metal. Preferably after formation of the binder and zeolite into a support, the support can be dried at a temperature of 50 - 320° C, preferably 100 - 200° C. for a period of 1 - 24 hours or more. Next, the support is usually calcined or oxidized at a temperature of 50 - 700° C., desirably 540 -650° C. for a period of 1 - 20 hours, desirably 1 -1.5 hours in an air atmosphere until the metallic compounds, if present, are converted substantially to the oxide form, and substantially all the alumina binder is converted to gamma-alumina. If desired, the optional halogen component may be adjusted by including a halogen or halogen-containing compound in the air atmosphere. The various heat treating steps may be conducted multiple times such as before and after addition of components, such as one or more metals, to the support via impregnation as is well known in the art. Steam may be present in the heat treating atmospheres during these steps. During calcination and/or other heat treatments to the catalyst, the pore size distribution of the alumina binder can be shifted to larger diameter pores. Thus, calcining the catalyst can increase the average pore size of the catalyst.

Optionally, the catalyst can be washed. Typically, the catalyst can be washed with a solution of ammonium nitrate or ammonium hydroxide, preferably ammonium hydroxide. Generally, the wash is conducted at a temperature of 50 - 150° C. for 1 - 10 hours. In one desired embodiment, no wash is conducted. Exemplary catalysts without a wash are depicted in US Pub. No. 2005/0143615 A1. Preferably, no wash or a wash of ammonium hydroxide is conducted to allow much of the existing alkali metal to remain on the catalyst.

In some exemplary embodiments, after drying, calcining, and optionally washing, one or more components can be impregnated on the support. The catalyst includes a noble metal, including one or more of platinum, palladium, rhodium, ruthenium, osmium, and iridium. The preferred noble metal is platinum. The noble metal component may exist within the final catalyst as a compound such as an oxide, sulfide, halide, or oxysulfide, or as an elemental metal or in combination with one or more other ingredients of the catalyst. Desirably, the noble metal component exists in a reduced state. This component may be present in the final catalyst in any amount which is catalytically effective. The final catalyst includes 0.1 - 2%, and optimally 0.1 - 0.5%, by weight, calculated on an elemental basis of the noble metal.

The noble metal component may be incorporated into the catalyst in any suitable manner. One method of preparing the catalyst involves the utilization of a water-soluble, decomposable compound of a noble metal to impregnate the calcined sieve-binder composite. Alternatively, a noble metal compound may be added at the time of compositing the sieve component and binder. Complexes of noble metals that may be employed in impregnating solutions, co-extruded with the sieve and binder, or added by other known method can include chloroplatinic acid, chloropalladic acid, ammonium chloroplatinate, bromoplatinic acid, platinum trichloride, platinum tetrachloride hydrate, platinum dichlorocarbonyl dichloride, tetramine platinic chloride, dinitrodiaminoplatinum, sodium tetranitroplatinate (II), palladium chloride, palladium nitrate, palladium sulfate, diaminepalladium (II) hydroxide, and tetraminepalladium (II) chloride.

A Group IVA (IUPAC 14) metal component may also be incorporated into the catalyst. Of the Group IVA (IUPAC 14) metals, germanium and tin are preferred and tin is especially preferred. This component may be present as an elemental metal, as a chemical compound such as the oxide, sulfide, halide, or oxychloride, or as a physical or chemical combination with the porous carrier material and/or other components of the catalyst. Preferably, a substantial portion of the Group IVA (IUPAC 14) metal exists in the finished catalyst in an oxidation state above that of the elemental metal. The Group IVA (IUPAC 14) metal component optimally is utilized in an amount sufficient to result in a final catalyst containing 0.01 - 5%, by weight, preferably 0.1 to 2%, by weight, and optimally 0.3 - 0.45, by weight, metal calculated on an elemental basis.

The Group IVA (IUPAC 14) metal component may be incorporated in the catalyst in any suitable manner to achieve a homogeneous dispersion, such as by co-precipitation with the porous carrier material, ion-exchange with the carrier material or impregnation of the carrier material at any stage in the preparation. One method of incorporating the Group IVA (IUPAC 14) metal component into the catalyst involves the utilization of a soluble, decomposable compound of a Group IVA (IUPAC 14) metal to impregnate and disperse the metal throughout the porous carrier material. The Group IVA (IUPAC 14) metal component can be impregnated either prior to, simultaneously with, or after the other components are added to the carrier material. Thus, the Group IVA (IUPAC 14) metal component may be added to the carrier material by commingling the latter with an aqueous solution of a suitable metal salt or soluble compound such as stannous bromide, stannous chloride, stannic chloride, stannic chloride pentahydrate; germanium oxide, germanium tetraethoxide, or germanium tetrachloride; or lead nitrate, lead acetate, or lead chlorate. The utilization of Group IVA (IUPAC 14) metal chloride compounds, such as stannic chloride, germanium tetrachloride or lead chlorate, is particularly preferred since it can facilitate the incorporation of both the metal component and at least a minor amount of the preferred halogen component in a single step. When combined with hydrogen chloride during the especially preferred alumina peptization step described hereinabove, a homogeneous dispersion of the Group IVA (IUPAC 14) metal component can be obtained. In an alternative embodiment, organic metal compounds such as trimethyltin chloride and dimethyltin dichloride are incorporated into the catalyst during the peptization of the alumina with hydrogen chloride or nitric acid.

The catalyst may also contain other metal components as well. Such metal modifiers may include rhenium, cobalt, nickel, indium, gallium, zinc, uranium, dysprosium, thallium, or a mixture thereof. Generally, a catalytically effective amount of such a metal modifier may be incorporated into a catalyst to effect a homogeneous or stratified distribution.

The catalyst can also contain a halogen component, such as fluorine, chlorine, bromine, iodine or a mixture thereof, with chlorine being preferred. Desirably, the catalyst contains no added halogen other than that associated with other catalyst components.

The catalyst also contains at least one alkali metal with a total alkali metal content of the catalyst of at least 200 ppm, by weight, calculated on an elemental basis. The alkali metal can be lithium, sodium, potassium, rubidium, cesium, francium, or a combination thereof. Preferred alkali metals can include sodium and potassium. Desirably, the catalyst contains no added alkali metal other than that associated with the zeolite and/or binder. The total alkali metal content of the catalyst is at least 200 ppm, desirably 300 ppm, by weight, calculated on an elemental basis. Generally, the total alkali metal content of the catalyst is no more than 2500 ppm, desirably 2000 ppm, and optimally 1000 ppm, by weight, calculated on an elemental basis. In one preferred embodiment, the catalyst can have 300 ppm - 2500 ppm, by weight of at least one alkali metal calculated on an elemental basis. In another preferred embodiment, the catalyst can have 150 - 250 ppm, preferably 200 - 250 ppm, by weight, sodium and at least 50 ppm, and preferably 150 - 250 ppm, by weight, potassium, calculated on an elemental basis.

The resultant catalyst can subsequently be subjected to a substantially water-free reduction step to ensure a uniform and finely divided dispersion of the optional metallic components. The reduction may be effected in the process equipment of the aromatic complex. Substantially pure and dry hydrogen (i.e., less than 100 vol. ppm, preferably 20 vol. ppm, $H_2O$) preferably is used as the reducing agent. The reducing agent can contact the catalyst at conditions, including a temperature of 200 - 650° C. and for a period of 0.5 - 10 hours, effective to reduce substantially all of the Group VIII metal component to the metallic state. In some cases, the resulting reduced catalyst may also be beneficially subjected to presulfiding by a known method such as with neat $H_2S$ at room temperature to incorporate in the catalyst in an amount of 0.05 - 1.0%, by weight, sulfur, calculated on an elemental basis.

The elemental analysis of the components of the catalyst, such as noble metal component and the at least one alkali metal can be determined by Inductively Coupled Plasma (ICP) analysis according to UOP Method 961-98.

Generally, catalysts described herein have several beneficial properties that provide isomerization of ethylbenzene while minimizing C8 ring-loss. Although not wanting to be bound by theory, such catalysts typically have one or more properties in common that provide isomerization of ethylbenzene while minimizing C8 ring-loss. As an example, the catalyst has a pore volume distribution of at least 70%, desirably 80%, of a catalyst pore volume defined by pores having a diameter greater than 100 Å and at least 95%, desirably 99%, of the catalyst pore volume defined by pores having a diameter less than 1000 Å. The pore volume is determined by nitrogen porsimetry using an instrument sold under the trade designation ASAP 405N by Micrometrics Instrument Corporation of Norcross, GA.

The procedure is derived from UOP-874-88, but uses 20 instead of 5 point pressure profiles. Generally, a sample is pre-treated at 300° C. for 16 hours in a vacuum at less than 0.0067 kPa. The pressure points relative to atmospheric pressure are: 0.05, 0.10, 0.15, 0.20, 0.25, 0.30, 0.35, 0.40, 0.45, 0.50, 0.55, 0.60, 0.65, 0.70, 0.75, 0.80, 0,85, 0.90, 0.96 and 0.97. From the data, the percentage of pores having a diameter greater or lesser than a given value can be determined by using the BJH adsorption model, see for example Barrett, E. P., Joyner, L. G. and Halenda, P. P., 1951, "The Determination of Pore Volume and Area Distributions in Porous Substances; I. Computations from Nitrogen Isotherms"; J. Amer. Chem. Soc. 73, 373-380. This data for four samples is depicted in FIG. 1 and discussed in greater detail hereinafter.

In addition, a catalyst described herein generally has a piece density of less than 1.250 g/cc, preferably of less than 0.950 g/cc, more preferably of less than 0.900 g/cc, and optimally 0.800 -0.890 g/cc as determined by mercury displacement according to UOP-766-91. Furthermore, the catalyst described herein generally has a surface area (may be referred herein as BET-SA) of at least 190 $m^2/g$, preferably at least 210 $m^2/g$, and optimally 220 - 250 $m^2/g$ as determined by UOP-874-88. All the UOP methods, such as UOP-766-91, UOP-874-88, and UOP-961-98, discussed herein can be obtained through ASTM International, 100 Barr Harbor Drive, West Conshohocken, PA, USA.

ILLUSTRATIVE EMBODIMENTS

The following examples are intended to further illustrate the subject catalyst. These illustrations of embodiments of the invention are not meant to limit the claims of this invention to the particular details of these examples. These examples are based on engineering calculations and actual operating experience with similar processes.

The exemplary catalysts can have a commercially synthesized MTW zeolite and an alumina source of either VERSAL-251 sold by UOP, LLC, an alumina sold under the trade designation CATAPAL C by Sasol North America of Houston, TX, or an aluminum hydroxychloride hydrosol (alsol or ODS alumina). All of these alumina sources can be converted to gamma alumina by heat treatment, yet they have different properties and performance. Although the VERSAL-251 (V-251) and CATAPAL C, which are both Boehmite aluminas are normally used to prepare extrudates, and the alsol is normally used to prepare oil dropped spheres, generally the ultimate catalyst shape is not determined by the alumina source. Spherical catalysts can be prepared from Boehmite alumina binders and oil dropped spheres may be formed into extrudates.

To form the extrudate supports, the alumina is usually at least partially peptized with a peptizing agent such as nitric acid. The zeolite can be mixed with the at least partially peptized alumina or may be mixed with the alumina prior to peptization. Afterwards, typically the alumina and MTW zeolite mixture is extruded into a cylinder or a tri-lobe shape. That being done, the extrudate can be dried and then calcined at 540 - 650° C. for 60 - 90 minutes.

The catalysts can be washed with ammonium nitrate or ammonium hydroxide, or not washed. If washed, the catalyst may be washed at a temperature of 90° C. for 5 hours. For an ammonium nitrate solution, the solution can include 1 g of ammonium nitrate and 5.7 g of water per gram of catalyst. For an ammonium hydroxide solution, 0.5%, by weight, of $NH_3$, in water can be used. The washing step may be conducted on the formed and calcined support prior to addition of the noble metal.

To form the oil-dropped support, an MTW zeolite is mixed with alsol. Generally, the alsol and MTW zeolite mixture is mixed with a gelling agent of hexamethylene tetraamine. Afterwards, the spheres can be formed and aged in the oil-dropping process. Next, generally the ODS supports may be washed with 0.5% ammonia, and calcined at 540 - 650° C. for 90 minutes.

The following can be undertaken for both the extruded supports and the ODS supports. Namely, all the supports can be impregnated with platinum with a solution of chloro-platinic acid mixed with water and HCl. Generally, the HCl is in amount of 2%, by weight, of the support, and the excess solution is evaporated.

Next, the supports can be oxidized or calcined at a temperature of 565° C. for 60 - 120 minutes in an atmosphere of 5 - 15 mol % of steam with a water to chloride ratio of 50:1 -120:1.

Afterwards, generally the supports are reduced at 565° C. for 120 minutes in a mixture of at least 15 mol% hydrogen in nitrogen. That being done, the supports can be sulfided in a 10 mol % atmosphere of hydrogen sulfide in a hydrogen sulfide and hydrogen mixture at ambient conditions to obtain 0.07%, by weight, sulfur on the support to obtain the final catalysts. A depiction of the materials and methods for forming the exemplary catalysts is provided in the table below:

**TABLE 1**

| Catalyst Example No. | Support Type | Shape | Forming Method | Alumina Source | Amount MTW (%, by weight) | Wash |
|---|---|---|---|---|---|---|
| A1 | IX'd CB | Trilobe | Extrusion | V-251 | 5 | $NH_4OH$ |
| A2 | CB | Trilobe | Extrusion | V-251 | 5 | None |
| A3 | IX'd CB | Trilobe | Extrusion | V-251 | 5 | $NH_4NO_3$ |
| A4 | IX'd CB | Cylindrical | Extrusion | V-251 | 5 | $NH_4NO_3$ |
| A5 | CB | Trilobe | Extrusion | V-251 | 10 | None |
| A6 | CB | Trilobe | Extrusion | V-251 | 10 | None |
| A7 | IX'd CB | Trilobe | Extrusion | V-251 | 10 | $NH_4NO_3$ |
| A8 | IX'd CB | Trilobe | Extrusion | V-251 | 10 | $NH_4OH$ |
| B1 | CB | Sphere | Oil Dropping | ODS | 5 | $NH_4OH$ |
| B2 | CB | Sphere | Oil Dropping | ODS | 5 | $NH_4OH$ |
| B3 | IX'd CB | Cylinder | Extrusion | ODS | 5 | $NH_4NO_3$ |
| C1 | IX'd CB | Cylinder | Extrusion | Catapal C | 5 | $NH_4NO_3$ |
| C2 | IX'd CB | Cylinder | Extrusion | Catapal C | 5 | $NH_4OH$ |

As used in the table, the term "CB" refers to a calcined base including the binder and zeolite, and the term "IX'd CB" refers to a calcined base washed with a solution of $NH_4OH$ or $NH_4NO_3$, as depicted in the "Wash" column for that row in TABLE 1.

The following data are depicted for the reduced catalysts in the following table. The LOI is conducted in accordance with UOP275-98. All components are provided in percent, by weight.

**TABLE 2**

| Catalyst Example No. | LOI @ 900ºC Weight% | Cl Weight% | Pt Weight% | Si Weight% | Na Weight% | K Weight% |
|---|---|---|---|---|---|---|
| A1 | 1.34 | 0.61 | 0.326 | 2.24 | 0.021 | 0.015 |
| A2 | 0.86 | 0.74 | 0.458 | 2.25 | 0.022 | 0.015 |
| A3 (comparative example) | 0.34 | 0.58 | 0.314 | 2.28 | 0.003 | <0.005 |
| A4 (comparative example) | 0.95 | 0.61 | 0.309 | 2.26 | 0.003 | <0.005 |
| A5 | 0.1 | 0.62 | 0.313 | 3.55 | 0.03 | 0.025 |
| A6 | 0.7 | 0.65 | 0.45 | 3.55 | 0.031 | 0.025 |
| A7 (comparative example) | 0.53 | 0.58 | 0.316 | 3.58 | 0.004 | <0.005 |
| A8 | 0.47 | 0.59 | 0.315 | 3.62 | 0.025 | 0.021 |
| B1 (comparative example) | 1.14 | 0.49 | 0.311 | 2.45 | 0.008 | <0.005 |
| B2 (comparative example) | 1.45 | 0.63 | 0.3 | 2.28 | 0.009 | <0.005 |
| B3 (comparative Example) | 1.4 | 0.57 | 0.324 | 2.1 | <0.002 | <0.005 |
| C1 (comparative example) | 1 | 0.67 | 0.32 | 2.24 | <0.002 | <0.005 |
| C2 (comparative example) | 0.58 | 0.61 | 0.308 | 2.24 | 0.014 | 0.012 |

Referring for FIG. 1, a pore distribution is depicted for reduced catalysts A1, B2, B3 and C1. As depicted, the exemplary catalyst made with a V-251 alumina exhibits a broader distribution of pores than the exemplary catalysts made with either ODS alumina or CATAPAL C alumina. Although not wanting to be bound by theory, it is believed that the broader distribution of pores in the reduced catalysts results in a catalyst for an isomerization process having less C8 ring loss.

More, the reduced catalysts are evaluated for several property measurements, as depicted below:

**TABLE 3**

| Catalyst Example No. | Piece Density (Volatile Free) g/cc | BET-SA square-meter/gram | % Pore Volume Greater Than 100 Å % |
|---|---|---|---|
| A1 | 0.868 | 230 | 81 |
| A2 | 0.869 | 247 | 72 |
| A3 (comparative example) | 0.869 | 223 | 83 |
| A4 (comparative example) | 0.839 | 226 | 83 |
| A5 | 0.874 | 242 | 81 |
| A6 | 0.872 | 239 | 81 |
| A7 (comparative example) | 0.886 | 237 | 81 |
| A8 | 0.879 | 242 | 80 |
| B1 (comparatives example) | 0.924 | 208 | 86 |
| B2 (comparatives example) | 0.969 | 208 | No Data |

(continued)

| Catalyst Example No. | Piece Density (Volatile Free) g/cc | BET-SA square-meter/gram | % Pore Volume Greater Than 100 Å % |
|---|---|---|---|
| B3 (comparatives example) | 0.909 | 193 | 88 |
| C1 (comparative example) | 1.209 | 216 | 61 |
| C2 (comparative example) | 1.203 | 207 | 65 |

Most of the catalysts from Table 2 are sulfided and evaluated for C8 aromatic ring loss using a pilot plant flow reactor processing a nan-equilibrium C8 aromatic feed having the following composition in perfect, by weight:

### TABLE 4

| Feed Composition | |
|---|---|
| Component | Percent, By Weight |
| Ethylbenzene | 14 |
| Para-xylene | <1 |
| Meta-xylene | 55 |
| Ortho-xylene | 22 |
| Toluene | 1 |
| C8 Paraffins | <1 |
| C8 Naphthenes | 6 |
| Water | 100 - 200 ppm |

This feed is contacted with a catalyst at a pressure of 700 kPa(g), a weight hourly space velocity (may be referred to as WHSV) of 8.0 $hr^{-1}$, and a hydrogen/hydrocarbon mole ratio of 4. The reactor temperature is 385° C.

The "C8 ring loss" is in mole percent as defined as "(1-(C8 naphthenes and aromatics in product )/(C8 naphthenes and aromatics in feed ))* 100", which represents a loss of one or more C8 rings that can be converted into a desired C8 aromatic, such as paraxylene. This loss of feed generally requires more feed to be provided to generate a given amount of product, reducing the profitability of the unit. Generally, a low amount of C8 ring loss is a favorable feature for a catalyst. The "C8 ring loss" (may be abbreviated herein as "C8RL") can be measured in the table below at conversion of the following formula:

$$pX/X * 100\% = 22.2 \pm 0.05\%$$

where:

pX represents moles of para-xylene in the product; and
X represents moles of xylene in the product.

Generally, the WHSV is set at 8 $hr^{-1}$ at the start of the test and is increased until pX/X * 100% = 22.2 $\pm$ 0.05%. Exemplary catalysts are tested in the pilot plant for C8 ring loss with the following results:

### TABLE 5

| Catalyst Example No. | Na + K PPM | C8RL Mole% | WHSV |
|---|---|---|---|
| A1 | 360 | 2.1 | 9.7 |
| A2 | 370 | 2.1 | 11 |

(continued)

| Catalyst Example No. | Na + K PPM | C8RL Mole% | WHSV |
|---|---|---|---|
| A3 (comparative examples) | <80 | 2.4 | 11.5 |
| A4 (comparative examples) | <80 | 2.6 | 9.7 |
| A5 | 550 | 2.3 | 17 |
| A6 | 560 | 2.3 | 17 |
| A7 (comparative examples) | <90 | 3.0 | 18 |
| A8 | 460 | 2.4 | 15 |
| B1 (comparative examples) | <130 | 2.6 | 10.7 |
| B2 (comparative examples) | <70 | 3.0 | 12.0 |
| C1 (comparative examples) | <70 | 3.4 | 10.2 |
| C2 (comparative examples) | 260 | 2.6 | 9.7 |

As depicted above, the C8 ring loss is compared with the total alkali metal content at a given WHSV. Catalysts having more than 200 ppm of sodium and potassium and derived from VERSAL-251 alumina have C8RL values ranging from 2.1 - 2.4 mole percent with an average C8RL of 2.2 mole percent. These values are substantially lower than the C8RL values for either catalysts derived from CATAPAL C alumina (ranging from 2.6 - 3.4 mole percent and averaging 3.0 mole percent) or an ODS alumina (ranging from 2.6 - 3.0 mole percent and averaging 2.8 mole percent).

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing, all temperatures are set forth uncorrected in degrees Celsius and, all parts and percentages are by weight, unless otherwise indicated.

From the foregoing description, one skilled in the art can easily ascertain the essential characteristics of this invention and, without departing from the spirit and scope thereof, can make various changes and modifications of the invention to adapt it to various usages and conditions.

## Claims

1. A catalyst for a C8 aromatic isomerization process, comprising:

    a) 1 - 10%, by weight, of a zeolite, wherein the zeolite is an MTW zeolite;
    b) 10 - 99%, by weight, of a binder, wherein the binder is a gamma-alumina, the gamma-alumina being derived from a Boehmite alumina;
    c) 0.1 - 2% weight, of a noble metal, calculated on an elemental basis; and
    d) at least one alkali metal wherein a total alkali metal content of the catalyst is at least 200 ppm, by weight, calculated on an elemental basis;
    e) wherein the catalyst has a pore volume distribution and at least 70% of a pore volume of the catalyst is defined by pores having a diameter greater than 100 Å, wherein the pore volume is determined as outlined in the description.

2. The catalyst according to claim 1, wherein the catalyst comprises 300 ppm - 2500 ppm, by weight, of the at least one alkali metal calculated on an elemental basis.

3. The catalyst according to any one of the preceding claims, wherein the at least one alkali metal comprises 200 - 250 ppm, by weight, of sodium and 150 - 250 ppm, by weight, of potassium, calculated on an elemental basis.

4. The catalyst according to any one of the preceding claims, wherein at least 80% of the pore volume of the catalyst is defined by pores having a diameter greater than 100 Å.

5. The catalyst according to any one of the preceding claims, wherein the catalyst has the shape of a trilobe extrudate.

**6.** The catalyst according to any one of the preceding claims, wherein the catalyst has the shape of a cylindrical extrudate.

**7.** The catalyst according to any one of the preceding claims, wherein the catalyst comprises 0.1 - 0.5%, by weight, of the noble metal, calculated on an elemental basis.

**8.** The catalyst according to any one of the preceding claims, wherein the noble metal comprises platinum.

**9.** The catalyst according to any one of the preceding claims, wherein at least 95% of the pore volume of the catalyst is defined by pores having a diameter less than 1000 Å.

**Patentansprüche**

**1.** Katalysator für ein C8-Aromaten-Isomerisierungsverfahren, umfassend:

a) 1-10 Gew.-% eines Zeoliths, wobei es sich bei dem Zeolith um einen MTW-Zeolith handelt;
b) 10-99 Gew.-% eines Bindemittels, wobei es sich bei dem Bindemittel um ein gamma-Aluminiumoxid handelt, wobei sich das gamma-Aluminiumoxid von einem Böhmit-Aluminiumoxid ableitet;
c) 0,1-2 Gew.-% eines Edelmetalls, berechnet auf Elementbasis; und
d) mindestens ein Alkalimetall, wobei ein Gesamtalkalimetallgehalt des Katalysators mindestens 200 Gew.-ppm, berechnet auf Elementbasis, beträgt;
e) wobei der Katalysator eine Porenvolumenverteilung aufweist und mindestens 70% eines Porenvolumens des Katalysators durch Poren mit einem Durchmesser von mehr als 100 Å definiert sind, wobei das Porenvolumen gemäß den Angaben in der Beschreibung bestimmt wird.

**2.** Katalysator nach Anspruch 1, wobei der Katalysator 300 Gew.-ppm - 2500 Gew.-ppm des mindestens einen Alkalimetalls, berechnet auf Elementbasis, umfasst.

**3.** Katalysator nach einem der vorhergehenden Ansprüche, wobei das mindestens eine Alkalimetall 200 - 250 Gew.-ppm Natrium und 150 - 250 Gew.-ppm Kalium, berechnet auf Elementbasis, umfasst.

**4.** Katalysator nach einem der vorhergehenden Ansprüche, wobei mindestens 80% des Porenvolumens des Katalysators durch Poren mit einem Durchmesser von mehr als 100 Å definiert sind.

**5.** Katalysator nach einem der vorhergehenden Ansprüche, wobei der Katalysator die Form eines trilobalen Extrudats aufweist.

**6.** Katalysator nach einem der vorhergehenden Ansprüche, wobei der Katalysator die Form eines zylindrischen Extrudats aufweist.

**7.** Katalysator nach einem der vorhergehenden Ansprüche, wobei der Katalysator 0,1 - 0,5 Gew.-% des Edelmetalls, berechnet auf Elementbasis, umfasst.

**8.** Katalysator nach einem der vorhergehenden Ansprüche, wobei das Edelmetall Platin umfasst.

**9.** Katalysator nach einem der vorhergehenden Ansprüche, wobei mindestens 95% des Porenvolumens des Katalysators durch Poren mit einem Durchmesser von weniger als 1000 Å definiert sind.

**Revendications**

**1.** Catalyseur pour un procédé d'isomérisation aromatique en C8, comprenant :

a) 1-10% en poids d'une zéolithe, où la zéolithe est une zéolithe de type MTW ;
b) 10-99% en poids d'un liant, où le liant est une gamme-alumine, la gamma-alumine étant dérivée d'une alumine de bohémite ;
c) 0,1-2% en poids d'un métal noble, calculé sur une base élémentaire ; et

d) au moins un métal alcalin, où une teneur en métal alcalin totale du catalyseur est d'au moins 200 ppm en poids, calculé sur une base élémentaire ;

e) **caractérisé en ce que** le catalyseur possède une distribution de volume de pores et au moins 70% d'un volume de pores du catalyseur est défini par des pores ayant un diamètre supérieur à 100 Å, où le volume de pores est déterminé tel qu'exposé dans la description.

2. Catalyseur selon la revendication 1, **caractérisé en ce que** le catalyseur comprend 300 ppm-2500 ppm en poids du au moins un métal alcalin, calculé sur une base élémentaire.

3. Catalyseur selon l'une quelconque des revendications précédentes, **caractérisé en ce que** le au moins un métal alcalin comprend 200-250 ppm en poids de sodium et 150-250 ppm en poids de potassium, calculé sur une base élémentaire.

4. Catalyseur selon l'une quelconque des revendications précédentes, **caractérisé en ce qu'**au moins 80% du volume de pores du catalyseur est défini par des pores ayant un diamètre supérieur à 100 Å.

5. Catalyseur selon l'une quelconque des revendications précédentes, **caractérisé en ce que** le catalyseur se trouve sous la forme d'un extrudat trilobé.

6. Catalyseur selon l'une quelconque des revendications précédentes, **caractérisé en ce que** le catalyseur se trouve sous la forme d'un extrudat cylindrique.

7. Catalyseur selon l'une quelconque des revendications précédentes, **caractérisé en ce que** le catalyseur comprend 0,1-0,5% en poids du métal noble, calculé sur une base élémentaire.

8. Catalyseur selon l'une quelconque des revendications précédentes, **caractérisé en ce que** le métal noble comprend du platine.

9. Catalyseur selon l'une quelconque des revendications précédentes, **caractérisé en ce qu'**au moins 95% du volume de pores du catalyseur est défini par des pores ayant un diamètre inférieur à 1000 Å.

# EP 2 047 906 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050143615 A
- US 6740788 B1
- US 3201491 A
- US 3832449 A
- US 4452769 A
- US 4537758 A
- US 6652832 B
- US 2620314 A
- US 20050143615 A1

**Non-patent literature cited in the description**

- **BARRETT, E. P. ; JOYNER, L. G. ; HALENDA, P. P.** The Determination of Pore Volume and Area Distributions in Porous Substances; I. Computations from Nitrogen Isotherms. *J. Amer. Chem. Soc.,* 1951, vol. 73, 373-380